# FASCICULE DE BREVET EUROPEEN

(11) **EP 1 107 767 B1**
(45) Date de publication et mention de la délivrance du brevet: **16.11.2005**
(21) Numéro de dépôt: 99940254.8
(22) Date de dépôt: 30.08.1999
(51) Int. Cl.: A61K 33/04, A61P 29/00

(54) **COMPOSITION CONTENANT DU SELENIUM POUR LE TRAITEMENT DU SYNDROME DE REPONSE INFLAMMATOIRE SYSTEMIQUE (SIRS)**
SELENIUM ENTHALTENDE ZUSAMMENSETZUNG ZUR BEHANDLUNG VON SYSTEMISCHEN ENTZÜNDUNGSSYNDROMEN
USE OF SELENIUM FOR TREATING SYSTEMIC INFLAMMATORY RESPONSE SYNDROME (SIRS), AND COMPOSITION FOR IMPLEMENTING SAID TREATMENT

(30) Priorité: 31.08.1998 FR 9810889
(43) Date de publication de la demande: 20.06.2001
(73) Titulaire: Forceville, Xavier, 77860 Saint Germain sur Morin (FR)
(72) Inventeur: FORCEVILLE, Xavier, F-77860 Saint Germain sur Morin (FR); VITOUX, Dominique, F-75014 Paris (FR)
(74) Mandataire: Catherine, Alain
(86) Numéro de dépôt international: PCT/FR1999/002066
(87) Numéro de publication internationale: WO 2000/012101

(56) Documents cités:
- WO-A-96/30007
- ZIMMERMANN T ET AL: "Selensubstitution bei Sepsispatienten. Eine prospektiv randomisierte Studie." MED KLIN, SEP 15 1997, 92 SUPPL 3 P3-4, XP002105157 GERMANY cité dans la demande
- GARTNER R. ET AL: "SELENSUBSTITUTION BEI SEPSISPATIENTEN" MEDIZINISCHE KLINIK(MED. KLIN.), 92/SUPPL. 3 (12-14), XP002105158 Germany cité dans la demande
- BORNER J ET AL: "Selensubstitution bei schweren entzundlichen chirurgischen Krankheitsbildern sowie Verbrennungen und Verbruhungen im Kindesalter." MED KLIN, SEP 15 1997, 92 SUPPL 3 P17-9, XP002105159 GERMANY cité dans la demande

## Description

La présente invention est relative à l'utilisation du sélénium pour le traitement de patients atteints d'un syndrome de réponse inflammatoire systémique (SIRS).

Elle est en outre relative à une composition pour la mise en oeuvre de ce traitement.

Le rôle du sélénium, en tant qu'oligo-élément intervenant dans de nombreuses réactions de l'organisme, est largement reconnu.

Ainsi, cet élément joue un rôle majeur dans le système antioxydant intracellulaire, en tant que composant de la glutathione peroxydase notamment. De plus, le sélénium semble jouer un rôle direct dans la régulation du processus inflammatoire.

Depuis les années 1970, la déficience en sélénium a été associée avec des cardiomyopathies sévères, en particulier trouvées dans des populations vivant dans des régions de Chine déficientes en sélénium. L'efficacité du sélénite de sodium sous forme orale, aussi bien d'un point de vue prophylactique que curatif vis-à-vis de ces maladies a été décrite.

Le rôle du sélénium dans des situations de stress oxydatif intense a été montré.

**VITOUX et al.** (1996, Therapeutic Uses of trace elements, Neve et al. ed., Plenum Press, New York, 127-131) ont relevé que la concentration en sélénium plasmatique diminue de manière importante chez les patients admis dans des unités de soins intensifs et présentant un syndrome de réponse inflammatoire systémique.

Néanmoins, aucune indication n'est donnée quant à l'utilisation du sélénium pour traiter de tels patients.

**ZIMMERMANN et al.** (1997, Medizinische Klinik, 92, 3-4 suppl. III) ont décrit de manière peu précise les résultats d'une étude sur l'effet du sélénite de sodium chez des patients atteints du syndrome de réponse inflammatoire systémique. Dans cette étude, les patients reçoivent tout d'abord une injection de 1000 µg de sélénite de sodium, puis 1000 µg de sélénite de sodium par jour par perfusion continue pendant vingt huit jours. Les auteurs considèrent comme optimale la dose de sélénium administrée.

Néanmoins aucune indication n'est donnée quant à la pathologie des patients traités. Il est simplement indiqué que ce sont des patients atteints de SIRS, dont certains avec une défaillance d'organe d'importance mal précisée. En outre, ZIMMERMANN et al. mentionnent que le groupe témoin présente une mortalité de 40%, ce qui est un chiffre important, au regard du type de patient traité et de la valeur de l'indice de gravité mentionné. Ces chiffres sont donc peu crédibles et, de plus, sont discordants avec les autres données générales de cet article. Il n'était donc pas possible de déduire de cet article quelles pathologies pouvaient être traitées par le sélénium.

GARTNER et al. (Med. Klinik, 1997, vol. 92, Suppl. 3, pp.12-14) décrit les résultats d'une étude clinique dans laquelle des patients atteints du syndrome de réponse inflammatoire systémique ont reçu par rapport aux témoins une dose additionnelle respectivement de 500 µg, 250 µg et 125 µg de sélénite de sodium, à raison d'une dose par jour pendant 3 jours.

BORNER et al. (Med. Klinik, 1997, vol. 92, Suppl.3, pp.17-19) décrit une étude clinique chez 34 enfants âgés de 1 à 16 ans affectés de maladies inflammatoires chirurgicales, telles que des brûlures étendues. L'apport exogène de sélénium est réalisé à raison de 200 µg de sélénium pentahydrate chez les patients d'un poids inférieur à 15 kg, d'environ 500 µg chez des patients ayant un poids compris entre 15 et 30 kg et d'environ 1000 µg chez des patients ayant un poids supérieur à 30 kg.

Les faibles doses de sélénium administrées aux patients inclus dans les études cliniques décrites par ZIMMERMANN, GARTNER et BORNER se justifient par les nombreux préjugés existant à l'encontre de la mise en oeuvre de doses plus fortes, dont il était généralement admis qu'elles étaient toxiques et présentaient des risques pour la vie du patient.

La demande PCT N° WO 96/30007 concerne l'utilisation de dérivés mercapto et séléno comme inhibiteurs de l'enzyme synthase de l'oxyde nitrique ou NO synthase. Ce document décrit uniquement l'activité d'inhibition *in vitro* de cette enzyme, de tels résultats ne pouvant pas être transposés par l'homme du métier afin de prévoir l'activité *in vivo* de tels composés, ni à fortiori les doses auxquelles ces composés seraient actifs *in vivo.*

D'autres études décrivant l'effet du sélénium sur diverses pathologies ont été publiées. Ainsi l'article de YA-JUN HU et al. (1997, Biological Trace Element Research, 56, 331-341) décrit l'utilisation du sélénium pour réduire la toxicité d'un produit anticancéreux, le cisplatine, chez des patients cancéreux. Les patients sont traités à des doses de 4 mg par jour de sélénium, sous forme kappa-sélénocarraghénane, par voie orale.

Certaines de ces études présentent des résultats discordants et obtenus sur des bases expérimentales peu convaincantes.

Ainsi, l'homme du métier était confronté à un grand nombre de documents indiquant que le sélénium pouvait être utilisé dans diverses pathologies, sans pour autant avoir de réelles certitudes quant à l'effet de cet oligo-élément, considéré comme toxique et pro-oxydant aux posologies utilisées dans certaines situations de stress oxydatif.

Or certaines pathologies relevant d'un syndrome de réponse inflammatoire systémique (SIRS) sont responsables d'une mortalité assez importante, principalement dans les unités de soins intensifs, et de défaillances viscérales sévères pouvant nécessiter des thérapeutiques de suppléance lourdes.

Il était donc nécessaire de mettre au point un traitement permettant de réduire cette mortalité et de réduire l'importance des défaillances viscérales associées.

Mais les patients présentant un syndrome de type SIRS sont des patients dans un état d'affaiblissement important, consécutif à une situation de stress oxydatif, et qui sont considérés comme peu susceptibles de résister à des doses de sélénium réputées comme toxiques, et de plus pro-oxydantes en elles-mêmes.

De ce fait, il n'était pas possible pour l'homme du métier d'extrapoler les résultats obtenus sur des patients présentant d'autres pathologies, aux patients présentant un syndrome de type SIRS et d'utiliser des doses de sélénium considérées comme toxiques et pro-oxydantes dans une situation de stress oxydatif.

La présente invention a montré qu'il était possible de réduire la mortalité et l'importance des défaillances viscérales notamment rénale, respiratoire, hématologique (coagulation), cardio-vasculaire, hépatique, gastro-intestinale et neurologique relevant d'un syndrome de réponse inflammatoire systémique (SIRS) en utilisant des doses de sélénium importantes, au regard de celles généralement considérées comme toxiques par l'homme du métier.

Il a été ainsi montré que l'on obtenait une grande efficacité dans le traitement des syndromes de type SIRS en traitant les patients par un médicament contenant durant les premiers jours du traitement une forte dose de sélénium, puis en abaissant cette dose, dans la suite du traitement.

La présente invention a donc pour objet l'utilisation d'au moins une molécule contenant du sélénium, dans une quantité correspondant à une dose quotidienne d'environ 2 à 80 mg, et préférentiellement 4 à 40 mg d'équivalent sélénium atomique, pour la fabrication d'un médicament pour le traitement d'un syndrome de réponse inflammatoire systémique (SIRS) sévère, ou de tout état correspondant à une poussée aiguë sévère de pathologie inflammatoire faisant intervenir une exacerbation de sécrétion de cytokines. Correspond notamment à cette définition tout état infectieux aigu sévère, que l'infection soit d'origine bactérienne, fongique, virale ou parasitaire.

Une dose de 2 à 80 mg d'équivalent sélénium atomique correspond à une dose de 0,025 à 1 mg/kg, qui est l'intervalle de dose préférentiel, chez l'homme ou l'animal. L'administration de telles doses implique un suivi clinique rigoureux.

Selon la présente invention on entend par syndrome de réponse inflammatoire systémique, ou SIRS, toute pathologie répondant à la définition donnée par **BONE et al** en 1992 lors de la conférence de normalisation de l'ACCP/SCCM (BONE et al, 1992, Chest, 101, 1644-1655).

L'invention est applicable en médecine humaine et vétérinaire.

De manière générale, le médicament administré lors de la phase initiale du traitement, préférentiellement le premier ou les quatre premiers jours du traitement, comprend une quantité de la molécule contenant du sélénium capable de réduire drastiquement l'état inflammatoire du patient durant cette phase initiale du traitement. Durant cette période particulièrement, le médicament est adapté pour l'administration d'une quantité de la ou des molécules contenant du sélénium suffisante pour maintenir l'état inflammatoire du patient ou de l'animal au-dessous d'un certain seuil. Ainsi, les quantités de la ou des molécules contenant du sélénium administrées quotidiennement peuvent être adaptées à la situation inflammatoire propre à chaque patient, le niveau de la réponse inflammatoire pouvant être vérifié pour chaque patient tout au long du traitement.

Par exemple, le niveau de l'état inflammatoire peut être évalué par la quantification de différentes cytokines dans le plasma, préférentiellement de I'IL-6 qui est le témoin considéré actuellement comme le plus fiable de l'importance d'une situation inflammatoire, mais aussi du TNF-α ou encore de l'IL-1

On évaluera préférentiellement la quantité d'interleukine-6 présente dans le sérum ou le plasma, par exemple, par un test de type ELISA tel que celui commercialisé par la Société MEDGENIX (Belgique).

La dose quotidienne de la ou des molécules contenant du sélénium sera adaptée de telle manière à maintenir un niveau d'interleukine-6 circulante inférieur d'au moins 30%, avantageusement d'au moins 40%, et de manière tout à fait préférée d'au moins 50% inférieur à la quantité d'interleukine-6 évaluée juste avant le traitement par une composition pharmaceutique selon l'invention. Ces valeurs sont données à titre indicatif et pourront varier selon la pathologie et, pour l'animal, selon l'espèce, en fonction des résultats cliniques obtenus avec la modulation de la réaction inflammatoire. Pour le suivi des taux d'interleukine-6 chez des patients dans une situation inflammatoire aiguë, l'homme du métier pourra avantageusement se référer à l'article de REINHART K. et al. (1996, crit. Care Med. Vol.24, n°5, pages 733-742).

Pour la quantification du taux de TNF-α circulant, l'homme du métier pourra se référer au test ELISA décrit par ENGELBERTS I. et al. (1991, Lancet, Vol. 338, pages 515-516).

La quantification d'IL-1 sérique ou plasmatique sera réalisée conformément à la technique décrite par MUNOZ C. et al. (1991, Eur. J. Immunol. vol.21, pages 2177-2184).

Le niveau de l'état de stress oxydatif d'un patient peut également être évalué par le test TBA-RS.

Enfin, le niveau des substances réactives à l'oxygène (ROS) constitue également un bon indicateur de l'état inflammatoire du patient dont la mesure peut être réalisée, par exemple, selon la technique décrite par FUKUYAMA N. (1997, vol. 22 (5), pages 771-774).

Il s'agit d'un test de mesure des concentrations des substances réagissant avec l'acide thiobarbiturique (TBARS), présentes à forte concentration, par exemple supérieure à 4 µ/mol/litre chez les patients dans une situation inflammatoire aiguë, cette valeur étant donnée à titre indicatif.

Pour mettre en oeuvre la mesure de la concentration des TBARS, l'homme du métier se référera avantageusement à l'article de GOODE H.F. et al. (1995, Critical Care Medicine, vol.28, n°4, pages 646-651).

Le niveau des peroxynitrites peut être apprécié par le dosage de la nitrotyrosine, voir « Cantitation of protein-band 3-nitrotyrosine and 3,4-dihydroxyphénylanine by high-performance liquid chromatography with electrochemical array detection » (HENSLEY K. Analytical Biochemistry 251, pages 187-195, 1997).

Le niveau de l'état inflammatoire du patient peut aussi être apprécié par une mesure de l'état de résistance à l'apoptose des polynucléaires, qui est un marqueur proposé de l'activation pro-inflammatoire de ces polynucléaires.

Pour effectuer cette mesure, l'homme du métier pourra se référer avantageusement à la technique décrite par MARTIN S.J. et al. (1996, Cell, vol. 82, page 349-352).

Le suivi de la situation inflammatoire du patient durant le traitement par une composition pharmaceutique selon l'invention peut également être effectué par une mesure de l'état d'activation du métabolisme oxydatif des polynucléaires neutrophiles, telle qu'une mesure par chimiluminescence comme décrit par exemple par ALLEN R.C. et al. (1986, Meth. Enzymol., vol.133, pages 449-493).

De manière préférentielle, le médicament correspondant à une dose quotidienne de 2 à 80 mg, et préférentiellement de 4 à 40 mg, d'équivalent sélénium atomique, est administré durant une courte période de temps, au début du traitement, la suite du traitement pouvant être mise en oeuvre avec des doses moins importantes de sélénium.

En conséquence, la présente invention a pour objet l'utilisation d'au moins une molécule contenant du sélénium, pour le traitement du SIRS dans une quantité correspondant à une dose quotidienne d'environ 2 à 80 mg d'équivalent sélénium atomique, au début du traitement, puis à une dose quotidienne de 0,5 à 2 mg d'équivalent sélénium atomique environ, dans la suite du traitement.

Selon un autre aspect, durant la période initiale du traitement, des doses, modulées selon la réaction inflammatoire, croissantes ou décroissantes de la ou des molécules contenant du sélénium, peuvent être administrées de manière à maintenir l'état inflammatoire du patient ou de l'animal au-dessous d'un certain niveau, et à un certain niveau, qui peut être vérifié à l'aide de l'une des techniques décrites précédemment. La ou les molécules contenant du sélénium peuvent ainsi être administrées à des doses quotidiennes variables et modulables durant la journée en fonction du contrôle de la réaction inflammatoire et du stress oxydatif allant de 2 à 80 mg d'équivalent sélénium atomique, soit de 0,025 mg/kg à 1 mg/kg d'équivalent sélénium atomique.

De manière préférentielle, un tel médicament est destiné au traitement des états de chocs septiques tels que péritonites, pneumopathies, méningites ou des septicémies bactériennes et, de manière plus générale, tout état inférieur aigu sévère mettant en jeu la vie du patient, que l'infection soit d'origine bactérienne, fongique, virale ou parasitaire.

Il est aussi destiné de manière générale au traitement de patients ayant une réaction immuno-inflammatoire sévère, liée à une pancréatite, une brûlure étendue, un polytraumatisme, une septicémie de quelque nature que ce soit, notamment bactérienne, mais aussi dans le cadre des états parasitaires fongiques ou viraux sévères, une intervention chirurgicale lourde, une intervention chirurgicale avec clampage (ischémie-reperfusion), un état de choc quel que soit son étiologie ou son type. Le nouveau médicament peut aussi être utilisé chez des patients présentant une défaillance viscérale. Le patient peut en outre être atteint d'une hépatopathie alcoolique, d'une cirrhose, quelle que soit son origine, d'une anorexie, d'une dénutrition, d'une malnutrition ou atteint du SIDA ou d'une pathologie inflammatoire chronique, notamment intestinale.

Selon un mode de mise en oeuvre préférentiel, le médicament est fabriqué afin d'aboutir à une dose quotidienne d'environ 2 à 80 mg, de préférence 4 à 40 mg, d'équivalent sélénium atomique, durant le premier jour, et éventuellement le deuxième, le troisième et le quatrième jours du traitement.

De manière tout aussi avantageuse il est fabriqué afin d'aboutir à une dose quotidienne d'environ 0,5 à 2 mg d'équivalent sélénium atomique pendant 1 à 20 jours, et, préférentiellement, de 1 à 10 jours durant la suite du traitement, soit 0,025 à 1 mg/kg et, de préférence, 0,05 à 0,5 mg/kg.

La molécule contenant du sélénium peut être toute molécule pharmacologiquement acceptable. Elle peut être un sel de sélénium, tel qu'un sélénite ou un sélénate du sélénium minéral, ou un sélénium organique, par exemple sélénocystéine, sélénométhionine, sélénodiglutathion, sélénométhyl sélénocystéine, diméthyl sélinoxyde, sélénocystamine, des levures séléniées ou des dérivés de synthèse chimique contenant un ou plusieurs atomes de sélénium. Elle est préférentiellement du sélénite de sodium.

Il est possible et parfois même avantageux, d'associer au cours de l'utilisation du sélénium telle que proposée par l'invention, des formes différentes de sélénium, en particulier durant la phase où de très fortes doses sont administrées (de 0,025 ou 0,05 à 1 mg/kg).

Le sélénite de sodium est la forme préférée mais d'autres formes de sélénium peuvent être utilisées en association, telles que la sélénocystéine, le sélénodiglutathion ou d'autres composés séléniés.

La mise en oeuvre d'un mélange de divers composés séléniés peut permettre de moduler plus spécifiquement tel ou tel aspect de la réaction de l'organisme au cours d'un SIRS sévère en tirant ainsi profit de l'effet d'un composé sélénié présent dans le mélange sur l'aspect spécifique à traiter: stress oxydatif, synthèse du NO, activation du NFKB et d'autres facteurs transcriptionnels, sécrétion de cytokines pro- et anti-inflammatoires, d'adhésines, activation de différentes cascades (acide arachidonique, coagulation, complément ...), activation des polynucléaires et des autres cellules phagocytaires, résistance initiale à l'apoptose de ces cellules phagocytaires, apoptose endothétiale et tissulaire viscérale secondaire. Par exemple, si le sélénite de sodium semble le plus approprié de façon générale, dans le contrôle de certaines composantes de la réaction inflammatoire systémique comme, notamment, pour moduler l'action sur l'apoptose, il est possible de lui associer d'autres composés séléniés.

Sans vouloir être lié par une quelconque théorie, le demandeur est d'avis que le sélénium agit sur des sites cibles tels que la glutathion peroxydase et la sélénoprotéine P (paroi des vaisseaux), de telle manière à diminuer drastiquement, aux quantités d'équivalent sélénium atomique quotidiennes selon l'invention, les effets délétères et le niveau des espèces réactives de l'oxygène (Reactive Oxygen Species ou ROS), et donc les conséquence, d'un stress oxydatif et le stress oxydatif excessif pour le patient ou l'animal. Le demandeur pense aussi que le sélénium permet une modulation de la concentration de peroxydes intracellulaires, notamment par son action sur la glutathion peroxydase, induisant une limitation de l'activation de certains facteurs de transcription, et notamment le facteur NFKB, ce qui pourrait conduire à une diminution de la production de NO synthase et de certaines cytokines telle que l'IL-6.

En outre, le demandeur pense, sans vouloir être lié par une telle théorie, que le sélénium à très fortes doses induit une apoptose significative de certaines cellules impliquées dans la réponse inflammatoire de l'hôte, notamment les polynucléaires neutrophiles ou par modification de leur cycle cellulaire. Ces actions sur ces cellules sont de nature à réduire considérablement l'état inflammatoire du patient ou de l'animal, du moins aux fortes doses quotidiennes d'équivalent sélénium atomique préconisées. A l'inverse, à doses plus modérées bien qu'encore élevées au regard des doses considérées comme utilisables actuellement, notamment dans une situation de stress oxydatif, le sélénium peut diminuer l'apoptose délétère des états inflammatoires sévères (cellules endothéliales, cellules tissulaires viscérales), ceci, en particulier, par la diminution du stress oxydatif extra- et intracellulaire qu'il réalise.

Egalement, lorsque les composés séléniés sont utilisés conformément à l'invention, ils peuvent exercer, lorsqu'ils sont administrés à très forte dose, une action directe antibactérienne (bactéricide), antiparasitaire, antivirale ou antifongique.

En conséquence, un médicament selon l'invention contiendra avantageusement, en association avec une quantité thérapeutiquement efficace de la (ou des) molécule(s) contenant du sélénium, une quantité thérapeutiquement efficace d'au moins un composé capable d'inhiber le métabolisme oxydatif ou de diminuer la réaction inflammatoire.

L'invention a donc en outre pour objet l'utilisation d'au moins une molécule de sélénium telle que définie précédemment, en association avec une quantité efficace d'au moins un composé non sélénié inhibiteur du métabolisme oxydatif ou s'opposant aux conséquences du stress oxydatif ou inhibiteur de la réaction inflammatoire.

Divers composés inhibiteurs du métabolisme oxydatif ou renforçant les défenses de l'organisme contre le stress oxydatif peuvent être utilisés, dans un médicament selon l'invention, en association avec au moins une molécule contenant du sélénium.

Selon un premier aspect, un médicament selon l'invention comprend, en association avec la ou les molécules contenant du sélénium, de la vitamine E, associée éventuellement à de la vitamine C, participant à la protection des membranes contre le stress oxydatif, un précurseur du glutathion, connu dans l'état de la technique, comme la N-acétyl cystéine, le glutathion régénérant la glutathion peroxydase sous sa forme réduite.

Selon un second aspect, le médicament contient un chélateur du fer, comme la desferioxamine, susceptible de diminuer la production de peroxydes. La desferioxamine est avantageusement présente dans le médicament pour une dose quotidienne comprise entre 5 et 100 mg/kg. Le médicament peut contenir également un chélateur du cuivre, pour exercer le même effet.

Selon un troisième aspect, un médicament selon l'invention contient, en association avec la ou les molécules contenant du sélénium, une quantité thérapeutiquement efficace de zinc ou de cuivre.

Selon un quatrième aspect, le chélateur du cuivre et le cuivre sont inclus séparément dans un médicament selon l'invention, pour une utilisation décalée dans le temps. Avantageusement, une association de la ou des molécules contenant du sélénium avec un chélateur du cuivre est utilisée en début de traitement, puis une association de la ou des molécules contenant du sélénium et le cuivre est mise en oeuvre pour la suite du traitement.

Un tel médicament peut comprendre, outre la ou les molécules contenant du sélénium, de la vitamine E, de la vitamine C ou du zinc, ou toute autre molécule possédant une action antioxydante, et présentant une compatibilité pharmacologique avec la molécule contenant du sélénium. L'addition de ces vitamines ou de ce métal permet de potentialiser l'effet du sélénium.

A titre indicatif, un médicament, ou une composition pharmaceutique, peut présenter une quantité de vitamine E associée éventuellement à de la vitamine C pour une dose quotidienne comprise entre 20 et 2000 mg pour chacune de ces vitamines.

Un médicament ou une composition pharmaceutique selon l'invention peut en outre contenir du zinc pour une dose quotidienne comprise entre 5 et 50 mg, ou tout autre oligo-élément essentiel.

Avantageusement, le médicament comprendra du cuivre pour une dose quotidienne comprise entre 1 et 10 mg/kg.

Avantageusement, le médicament comprendra de la N-acétyl cystéine pour une dose quotidienne comprise entre 50 et 500 mg/kg/j.

De préférence, le médicament comprendra un composé inhibiteur de la réaction inflammatoire, par exemple de l'or pour une dose quotidienne comprise entre 25 et 300 mg/kg.

En cas d'insuffisance rénale, l'administration d'un chétateur à élimination urinaire, comme la desferioxamine, sera préférentiellement combinée à une épuration extra-rénale par hémodialfiltration continue ou encore par hémodialyse prolongée.

Selon un cinquième aspect, un médicament selon l'invention comprend plusieurs composés choisis parmi les composés inhibiteurs du métabolisme oxydatif et les composés diminuant ou inhibant la réaction inflammatoire.

De manière préférentielle, le médicament est mis sous une forme pharmaceutique injectable, perfusable ou pour administration entérale. Il peut être mis néanmoins sous toute autre forme permettant l'administration de la ou des molécules contenant du sélénium, et un traitement efficace du SIRS.

Ce médicament peut être administré par voie parentérale, de préférence intraveineuse, également sous-cutanée, intramusculaire, ainsi qu'également par voie intrapéritonéale, entérale ou orale.

Ce médicament est préférentiellement destiné à un traitement curatif. Il peut néanmoins être administré de manière préventive, en particulier avant une intervention chirurgicale lourde, notamment de chirurgie vasculaire, afin de limiter le stress oxydatif.

Un tel médicament ou composition pharmaceutique peut contenir, outre la ou les molécules contenant du sélénium, des excipients pharmaceutiquement compatibles. Sous la forme d'une perfusion, il peut comprendre entre environ 1,3 mg/l et 800 mg/l d'équivalent sélénium atomique.

La présente invention est illustrée, sans pour autant être limitée, par les exemples qui suivent.

### EXEMPLE 1

Un patient de 51 ans, 75 kg, éthylique chronique sans antécédent de décompensation ictéroascitique, hémorragique ou encéphalopathique, est admis en réanimation en postopératoire d'une péritonite purulente généralisée sur perforation colique au décours d'une poussée de sigmoïdite diverticulaire.

Son état hémodynamique initial est conservé sous remplissage. Il est intubé-ventilé sous sédation avec une FiO₂, un peu augmentée, à 50%. Il existe une insuffisance rénale modérée. Une antibiothérapie empirique adaptée a été débutée, antibiothérapie qui sera modifiée à 48 heures au vu des antibiogrammes. A 24 heures ses indices de gravité sont IGS II 29, APACHE II 17 et le SOFA score est à 5. Un jour après l'opération, le tableau s'aggrave rapidement avec installation d'un état de choc avec une acidose lactique à 5 µmol/l nécessitant la mise sous DOPAMINE puis rapidement sous NORADRENALINE jusqu'à 4 mg/h (soit 0,9 µg/kg/min). Il existe une aggravation de son état respiratoire nécessitant l'augmentation de la FiO₂ en raison de l'installation d'un syndrome de détresse respiratoire aiguë de l'adulte (SDRAA). Dès que la nécessité d'administration de NORADRENALINE a été ressentie, un traitement par du sélénite de sodium en administration continue a été débuté à raison de 4 mg d'équivalent sélénium atomique sur les 24 premières heures, suivi d'une administration continue de sélénite de sodium à raison de 1 mg d'équivalent sélénium atomique pendant 10 jours.

Ce traitement a permis de limiter l'importance de cet état de choc vasoplégique, évitant de ce fait un décès précoce. De même ce traitement a permis de limiter l'importance des défaillances viscérales. L'évolution a été marquée par la survenue d'une insuffisance rénale à diurèse conservée, mais ne nécessitant pas de dialyse. Une ventilation à FiO₂ 70% a été très transitoirement nécessaire en raison d'un SDRAA rapidement résolutif. L'administration de NORADRENALINE a été progressivement arrêtée en trois jours. L'acidose lactique a rapidement régressé. Il n'est pas survenu de coagulation intravasculaire disséminée, le taux de plaquettes est toujours resté supérieur à 150000 plaquettes/mm³. En postopératoire aucun épisode d'infection nosocomial ne sera constaté, en particulier il n'est pas survenu de pneumopathie nosocomiale. Il n'est pas survenu non plus de complication abdominale. Ce patient a quitté la réanimation 10 jours après l'opération.

Il est revu en consultation 3 mois après. Il a alors repris son activité professionnelle et son mode de vie habituel.

### EXEMPLE 2:

Une patiente de 35 ans, dépressive, anorexique, 51 kg pour 1m 75, est admise pour un diagnostic de tentative de suicide médicamenteuse avec ingestion d'une grande quantité d'analgésique et de sédatifs. Le diagnostic est rapidement redressé pour une péritonite purulente généralisée par perforation gastrique sur ulcère. Elle est transférée en réanimation en postopératoire. Il existe d'emblée un état de choc nécessitant un remplissage et l'introduction de catécholamines par NORADRENALINE et DOBUTAMINE; acidose lactique à 6 µmol/l. Une antibiothérapie à visée antibactérienne et antifongique est poursuivie. La diurèse est maintenue sous diurétiques. Une heure après le début de l'administration de NORADRENALINE, un traitement par du sélénite de sodium en administration continue a été débuté à raison de 4 mg d'équivalent sélénium sur les 24 premières heures, suivi d'une administration continue de sélénite de sodium à raison de 1 mg d'équivalent sélénium/j pendant 10 jours. A la 24ème heure la valeur de ses indices de gravité seront IGS Il 44, APACHE Il 35. Le SOFA score sera de 8.

L'évolution est favorable initialement avec régression de l'état de choc en 24 heures. Pas de défaillances viscérales importantes, reprise de la diurèse, (clearance de la créatinine à 40), ventilation en FiO₂ 60%, sans PEP (pression expiratoire positive), pas de trouble important de la coagulation en dehors d'un TP à 50%. Survenue de deux épisodes d'atélectasie nécessitant une fibroaspiration. Une alimentation entérale précoce a été instituée.

Huit jours après l'opération on observe la persistance d'un écoulement purulent par les drains. Au scanner abdominal il existe une collection sous-hépatique, sans épanchement péritonéal libre. Une ponction sous scanner permettra de drainer cette collection. Mise en évidence à la bactériologie, sur du pus franc, de colonies d'Hafnia alvei et de Candida albicans; adaptation de l'antibiothérapie à l'antibiogramme.

Douze jours après l'opération, une pneumopathie nosocomiale à Streptocoque alpha hémolytique survient (diagnostic par fibroscopie avec réalisation d'une brosse télescopique protégée et d'un lavage bronchoalvéolaire). Une antibiothérapie empirique à visée cocci gram positif est instaurée, puis adaptée à l'antibiogramme. L'extubation sera réalisée 20 jours après l'opération. Une kinésithérapie prolongée sera nécessaire pour éviter une réintubation.

Cette patiente est transférée en centre de convalescence pour poursuite de la rénutrition au 35 ème jour. Elle est revue en consultation à 3 mois. On observe une reprise de poids, avec un poids de 56 kg. Une psychothérapie a été débutée.

### EXEMPLE 3

Un patient de 57 ans, présentant une intoxication alcoolo-tabagique importante (vin plus d'un litre par jour, tabac 2 paquets par jour), une BPCO insuffisance respiratoire chronique, une artérite des membres inférieurs stade Il et une altération de l'état général depuis plusieurs mois avec une toux productive, est transféré en réanimation après un court séjour en médecine. Il existe à son admission une détresse respiratoire nécessitant une intubation-ventilation de sauvetage. Les gaz du sang confirmeront l'acidose respiratoire majeure. Ce patient est fébrile. Il existe une hyperdeucocytose à 24000 leucocytes dont 88% de polynucléaires. La tension est stable sous remplissage, toutefois on observe des marbrures des genoux. Il n'existe pas de trouble de la coagulation ni d'insuffisance rénale. A la 24ème heure l'IGS Il est de 41, l'APACHE Il de 26, et le SOFA score à 8. Les prélèvements pulmonaires par brosse télescopique protégée et lavage broncho-alvéolaire confirmeront le diagnostic de pneumopathie communautaire: 47% de cellules infectées, Haemophilus influenza ß-lactamase négatif et streptocoque anginosis sauvage. Une bi-antibiothérapie a été instituée immédiatement qui s'avérera être efficace sur ces germes. Au scanner thoraco-abdominal, il existe une volumineuse collection liquidienne au sein du parenchyme pulmonaire de la base droite, semblant se fistuliser en pleural avec une pleurésie. Par ailleurs, le scanner abdominal constate l'existence d'un anévrisme thrombosé de l'aorte abdominal sous rénal.

L'évolution immédiate est marquée par l'aggravation rapide de son état respiratoire avec nécessité d'une ventilation en FiO₂ 100%, PEP8. Par ailleurs, un remplissage très important est nécessaire sous couvert de mesure de pression par cathétérisme droit. De la DOPAMINE doit être introduite à raison de 10 µg/kg/min. Au bout de 8 heures d'administration de DOPAMINE, un traitement par du sélénite de sodium en administration continue a été débuté à raison de 4 mg d'équivalent sélénium sur les 24 premières heures, suivi d'une administration continue de sélénite de sodium à raison de 1 mg d'équivalent sélénium par jour pendant 10 jours.

Après augmentation de la DOPAMINE à 20 µg/kg/min et adjonction d'ADRENALINE à 1 mg/h, le tableau hémodynamique semble alors se stabiliser. L'hyperlactatémie augmente parallèlement jusqu'à 10 µmol/l, puis diminue à partir du deuxième jour. L'évolution vers un état de choc fatal peut ainsi être évité. Sur le plan respiratoire, un traitement par monoxyde d'azote (NO) est débuté à 10 ppm. La diurèse est maintenue sous diurétique. Il existe une thrombopénie à 7500 plaquettes/mm³ associée à un allongement des temps de coagulation et une augmentation des PDF (produits de dégradation de la fibrine) témoignant d'une CIVD modérée. Un drainage de la pleurésie purulente a été institué.

A partir du deuxième jour on constate une amélioration progressive du tableau tant sur le plan respiratoire qu'hémodynamique. Le drainage permet une évacuation complète de la pleurésie avec un drainage de l'abcès pulmonaire. Le sevrage en catécholamine est obtenu au cinquième jour. L'extubation est réalisée au dixième jour. Ce patient sera retransféré au quinzième jour en pneumologie pour la poursuite de l'exploration et de la prise en charge de son insuffisance respiratoire.

Ce patient est revu en consultation 3 mois après. Un abcès dentaire a été traité. Une réinsertion professionnelle est en cours. Ce patient n'a pas d'oxygénation à domicile.

Ces résultats sont concordants avec ceux obtenus sur une grande série, montrant une amélioration nette du pronostic des patients traités par du sélénium à forte dose par rapport à ceux ayant reçu un placebo.

On entend par IGS II, l'indice de gravité simplifié II défini par **LE GALL et al.** en 1993 (A New Simplified Acute Physiology Score [SAPS II] Based on a European/North American Multicenter Study. JAMA, 1993; 270:2957-2963), par APACHE II, (Acute Physiology and Chronic Health Evaluation II) l'indice de gravité défini par W.A. KNAUS et al. (APACHE II: A severity of disease classification system. Crit. Care Med. 1985; 13: 818-829), et par SOFA score, le score de défaillance viscérale défini par **JL VINCENT et al.** (The SOFA [Sepsis-related Organ Failure Assessment] score to describe organ dysfunction/failure. Intensive Care Med. 1995; 22:707-710).

## Revendications

1. Utilisation d'au moins une molécule contenant du sélénium, dans une quantité correspondant à une dose quotidienne d'environ 2 à 80mg d'équivalent sélénium atomique, soit d'environ 0,025 à 1 mg/kg, pour la fabrication d'un médicament pour le traitement du syndrome de réponse inflammatoire systémique sévère ou de tout état correspondant à un e poussée aiguë sévère de pathologie inflammatoire faisant intervenir une exacerbation de sécrétion de cytokines, ladite molécule contenant du sélénium étant choisie parmi un sel de sélénium, la sélénoprotéine P, la sélénocystéine, la sélénométhionine, le sélénoglutathion, la sélénométhyl sélénocystéine, le diméthylsélinoxyde, la sélénocystamine et des levures séléniées.

2. Utilisation d'au moins une molécule contenant du sélénium pour la fabrication d'un médicament pour le traitement du syndrome de réponse inflammatoire systémique sévère, dans une quantité correspondant à une dose quotidienne de 2 à 80 mg environ d'équivalent sélénium atomique, soit d'environ 0,025 à 1 mg/kg, du début du traitement, puis à une dose quotidienne d'environ 0,5 à 2 mg d'équivalent sélénium atomique, ladite molécule contenant du sélénium étant choisie parmi un sel de sélénium, la sélénoprotéine P, la sélénocystéine, la sélénométhionine, le sélénoglutathion, la sélénométhyl sélénocystéine, le diméthylsélinoxyde, la sélénocystamine et des levures séléniées.

3. Utilisation selon l'une des revendications 1 et 2, **caractérisée en ce que** le sel de sélénium est choisi parmi un sélénite ou un sélénate du sélénium minéral.

4. Utilisation selon l'une des revendications 1 à 3 dans laquelle le médicament est destiné au traitement d'états infectieux aigus sévères, tels que des péritonites, des pneumopathies, des méningites et des septicémies bactériennes en état de choc septique.

5. Utilisation selon l'une des revendications 1 à 3 pour le traitement d'états infectieux sévères qu'ils soient d'origine bactérienne, parasitaire, fongique ou virale et, de façon générale, toute affection s'accompagnant d'une réaction immuno-inflammatoire importante avec notamment élévation des cytokines circulantes, mais aussi plus localisée telle la polyarthrite rhumatoïde en poussée.

6. Utilisation du médicament selon l'une quelconque des revendications précédentes pour le traitement de l'homme ou de l'animal, les doses par kg étant, chez l'animal, modulées selon la dose léthale 50% (DL 50) de l'espèce par rapport à celle de l'espèce humaine.

7. Utilisation selon l'une quelconque des revendications précédentes dans laquelle le médicament est fabriqué afin d'aboutir à une dose quotidienne de 2 à 80 mg environ d'équivalent sélénium atomique, soit d'environ 0,025 à 1 mg/kg, durant le premier jour et éventuellement le deuxième, le troisième et le quatrième jour du traitement.

8. Utilisation selon l'une quelconque des revendications précédentes dans laquelle le médicament est fabriqué afin d'aboutir à une dose quotidienne d'environ 0,5 à 2 mg d'équivalent sélénium atomique, pendant de 1 à 20 jours durant la suite du traitement.

9. Utilisation selon l'une quelconque des revendications précédentes selon laquelle une des molécules contenant du sélénium est le sélénite de sodium.

10. Utilisation selon l'une quelconque des revendications précédentes dans laquelle plusieurs molécules contenant du sélénium sont utilisées simultanément pour moduler plus précisément différents compartiments de la réaction inflammatoire systémique.

11. Utilisation selon l'une quelconque des revendications précédentes **caractérisée en ce que** le médicament est sous une forme administrable par voie parentérale, de préférence intraveineuse, et également sous-cutanée, intramusculaire ainsi que intrapéritonéale, entérale ou orale, et avantageusement sous une forme pharmaceutique injectable ou perfusable ou à administration entérale.

12. Utilisation selon l'une quelconque des revendications précédentes, **caractérisée en ce que** le médicament contient au moins un composé associé non sélénié inhibiteur, ou diminuant les conséquences, du métabolisme oxydatif ou inhibiteur de la réaction inflammatoire.

13. Utilisation selon la revendication 12, **caractérisée en ce que** le composé associé non sélénié inhibiteur du métabolisme oxydatif est choisi parmi un précurseur du glutathion, un chélateur du fer, un chélateur du cuivre, du cuivre, du zinc, la vitamine E et éventuellement la vitamine C.

14. Utilisation selon la revendication 12, **caractérisée en ce que** le composé inhibiteur de la réaction inflammatoire est l'or.

15. Utilisation selon l'une quelconque des revendications précédentes, **caractérisée en ce que** le médicament contient un oligo-élément essentiel autre que le sélénium ou ceux énoncés précédemment (Cu, Zn).

16. Composition pharmaceutique **caractérisée en ce qu'**elle comprend une quantité de molécule(s) contenant du sélénium correspondant à une dose quotidienne d'environ 2 à 80 mg d'équivalent sélénium atomique soit d'environ 0,025 à 1 mg/kg, et des excipients pharmaceutiques compatibles, ladite molécule contenant du sélénium étant choisie parmi un sel de sélénium, la sélénoprotéine P, la sélénocystéine, la sélénométhionine, le sélénoglutathion, la sélénométhyl sélénocystéine, le diméthylsélinoxyde, la sélénocystamine et des levures séléniées.

17. Composition pharmaceutique selon la revendication 16, **caracterisée en ce que** le sel de sélénium est choisi parmi un sélénite ou un sélénate du sélénium minéral.

18. Composition pharmaceutique selon l'une des revendications 16 et 17, **caractérisée en ce qu'**elle contient au moins un composé associé non sélénié inhibiteur ou diminuant les conséquences du métabolisme oxydatif ou inhibiteur de la réaction inflammatoire.

19. Composition pharmaceutique selon la revendication 18, **caractérisée en ce que** le composé associé non sélénié est choisi parmi la vitamine E et éventuellement la vitamine C, un précurseur du glutathion, un chélateur du fer, un chélateur du cuivre, du cuivre, ou du zinc.

20. Composition pharmaceutique selon l'une des revendications 16 et 17, **caractérisée en ce que** le composé inhibiteur de la réaction inflammatoire est de l'or.

21. Composition selon l'une quelconque des revendications 16 à 20. **caractérisée en ce qu'**elle contient un oligo-élément essentiel autre que le sélénium ou ceux cités précédemment (Zn, Cu).

22. Composition selon l'une quelconque des revendications 16 à 21, **caractérisée en ce qu'**elle est sous une forme injectable, perfusable, à administration parentérale, préférentiellement intraveineuse (également sous-cutanée ou intramusculaire), mais aussi intrapéritonéale, entérale ou orale.

23. Composition selon l'une quelconque des revendications 16 à 21, **caractérisée en ce qu'**elle est sous la forme d'une perfusion comprenant entre environ 1,3 et 800 mg d'équivalent sélénium atomique par litre.

## Patentansprüche

1. Verwendung wenigstens eines Moleküls, das Selen enthält, in einer Menge, die einer Tagesdosis von ungefähr 2 bis 80 mg Atomäquivalent Selen entspricht, also ungefähr 0,025 bis 1 mg/kg, für die Herstellung eines Medikaments zur Behandlung von schwerem systemischem inflammatorischem Response-Syndrom (SIRS; Ganzkörperentzündungssyndrom) oder jedes Zustands, der einem schweren akuten Ausbruch einer Entzündungspathologie entspricht und zu einer Exazerbation der Cytokinausschüttung führt, wobei das Selen enthaltende Molekül aus einem Selensalz, Selenoprotein P, Selenocystein, Selenomethionin, Selenoglutathion, Selenomethylselenocystein, Dimethylselenoxid, Selenocystamin und selenierten Hefen ausgewählt ist.

2. Verwendung wenigstens eines Moleküls, das Selen enthält, für die Herstellung eines Medikaments zur Behandlung von schwerem systemischem inflammatorischem Response-Syndrom in einer Menge, die einer Tagesdosis von ungefähr 2 bis 80 mg Atomäquivalent Selen, also ungefähr 0,025 bis 1 mg/kg, am Anfang der Behandlung und dann einer Tagesdosis von ungefähr 0,5 bis 2 mg Atomäquivalent Selen entspricht, wobei das Selen enthaltende Molekül aus einem Selensalz, Selenoprotein P, Selenocystein, Selenomethionin, Selenoglutathion, Selenomethylselenocystein, Dimethylselenoxid, Selenocystamin und selenierten Hefen ausgewählt ist.

3. Verwendung gemäß einem der Ansprüche 1 und 2, **dadurch gekennzeichnet, dass** das Selensalz aus einem mineralischen Selenit oder Selenat ausgewählt ist.

4. Verwendung gemäß einem der Ansprüche 1 bis 3, wobei das Medikament zur Behandlung von schweren akuten infektiösen Zuständen dient, wie Peritonitis, Pneumopathie, Meningitis und bakterieller Sepsis im Zustand des septischen Schocks.

5. Verwendung gemäß einem der Ansprüche 1 bis 3 zur Behandlung von schweren infektiösen Zuständen, die auf Bakterien, Parasiten, Pilze oder Viren zurückgehen, und allgemein von allen Beschwerden, die mit einer starken immunoinflammatorischen Reaktion, insbesondere mit einer Erhöhung der zirkulierenden Cytokine, aber auch stärker lokalisiert, wie einem Ausbruch von rheumatoider Arthritis (chronischer Polyarthritis), einhergehen.

6. Verwendung des Medikaments gemäß einem der vorstehenden Ansprüche zur Behandlung von Mensch oder Tier, wobei die Dosen pro kg bei einem Tier gemäß der zu 50% letalen Dosis (LD₅₀) der Spezies im Verhältnis zu derjenigen beim Menschen moduliert werden.

7. Verwendung gemäß einem der vorstehenden Ansprüche, wobei das Medikament so hergestellt wird, dass man zu einer Tagesdosis von ungefähr 2 bis 80 mg Atomäquivalent Selen, also ungefähr 0,025 bis 1 mg/kg, während des ersten Tages und gegebenenfalls des zweiten, dritten und vierten Tages der Behandlung gelangt.

8. Verwendung gemäß einem der vorstehenden Ansprüche, wobei das Medikament so hergestellt wird, dass man zu einer Tagesdosis von ungefähr 0,5 bis 2 mg Atomäquivalent Selen während 1 bis 20 Tagen bei der anschließenden Behandlung gelangt.

9. Verwendung gemäß einem der vorstehenden Ansprüche, wobei es sich bei einem der Moleküle, die Selen enthalten, um Natriumselenit handelt.

10. Verwendung gemäß einem der vorstehenden Ansprüche, wobei mehrere Moleküle, die Selen enthalten, gleichzeitig verwendet werden, um verschiedene Kompartimente der systemischen inflammatorischen Reaktion genauer zu modulieren.

11. Verwendung gemäß einem der vorstehenden Ansprüche, **dadurch gekennzeichnet, dass** das Medikament in einer Form zur parenteralen, vorzugsweise intravenösen, und auch subkutanen, intramuskulären sowie intraperitonealen, enteralen oder oralen Verabreichung und vorteilhafterweise in einer injizierbaren oder perfundierbaren oder enteral verabreichbaren pharmazeutischen Form vorliegt.

12. Verwendung gemäß einem der vorstehenden Ansprüche, **dadurch gekennzeichnet, dass** das Medikament wenigstens eine nichtselenierte Begleitverbindung enthält, die den oxidativen Stoffwechsel hemmt oder dessen Folgen lindert oder die Entzündungsreaktion hemmt.

13. Verwendung gemäß Anspruch 12, **dadurch gekennzeichnet, dass** die nichtselenierte Begleitverbindung, die den oxidativen Stoffwechsel hemmt, aus einem Glutathion-Vorläufer, einem Eisenchelator, einem Kupferchelator, Kupfer, Zink, Vitamin E und gegebenenfalls Vitamin C ausgewählt ist.

14. Verwendung gemäß Anspruch 12, **dadurch gekennzeichnet, dass** es sich bei der Verbindung, die die Entzündungsreaktion hemmt, um Gold handelt.

15. Verwendung gemäß einem der vorstehenden Ansprüche, **dadurch gekennzeichnet, dass** das Medikament ein essentielles Oligoelement enthält, das von Selen oder den zuvor genannten (Cu, Zn) verschieden ist.

16. Pharmazeutische Zusammensetzung, **dadurch gekennzeichnet, dass** sie eine Menge eines oder mehrerer Selen enthaltender Moleküle, die einer Tagesdosis von ungefähr 2 bis 80 mg Atomäquivalent Selen entspricht, also ungefähr 0,025 bis 1 mg/kg, sowie pharmazeutisch annehmbare Arzneimittelhilfsstoffe umfasst, wobei das Selen enthaltende Molekül aus einem Selensalz, Selenoprotein P, Selenocystein, Selenomethionin, Selenoglutathion, Selenomethylselenocystein, Dimethylselenoxid, Selenocystamin und selenierten Hefen ausgewählt ist.

17. Pharmazeutische Zusammensetzung gemäß Anspruch 16, **dadurch gekennzeichnet, dass** das Selensalz aus einem mineralischen Selenit oder Selenat ausgewählt ist.

18. Pharmazeutische Zusammensetzung gemäß einem der Ansprüche 16 und 17, **dadurch gekennzeichnet, dass** sie wenigstens eine nichtselenierte Begleitverbindung enthält, die den oxidativen Stoffwechsel hemmt oder dessen Folgen lindert oder die Entzündungsreaktion hemmt.

19. Pharmazeutische Zusammensetzung gemäß Anspruch 18, **dadurch gekennzeichnet, dass** die nichtselenierte Begleitverbindung aus Vitamin E und gegebenenfalls Vitamin C, einem Glutathion-Vorläufer, einem Eisenchelator, einem Kupferchelator, Kupfer oder Zink ausgewählt ist.

20. Pharmazeutische Zusammensetzung gemäß einem der Ansprüche 16 und 17, **dadurch gekennzeichnet, dass** es sich bei der Verbindung, die die Entzündungsreaktion hemmt, um Gold handelt.

21. Zusammensetzung gemäß einem der Ansprüche 16 bis 20, **dadurch gekennzeichnet, dass** sie ein essentielles Oligoelement enthält, das von Selen oder den zuvor genannten (Zn, Cu) verschieden ist.

22. Zusammensetzung gemäß einem der Ansprüche 16 bis 21, **dadurch gekennzeichnet, dass** sie in einer injizierbaren, perfundierbaren, parenteral, vorzugsweise intravenös (auch subkutan oder intramuskulär), aber auch intraperitoneal, enteral oder oral verabreichbaren Form vorliegt.

23. Zusammensetzung gemäß einem der Ansprüche 16 bis 21, **dadurch gekennzeichnet, dass** sie in Form einer Perfusion vorliegt, die zwischen ungefähr 1,3 und 800 mg Atomäquivalent Selen pro Liter umfasst.

## Claims

1. Use of at least one molecule comprising selenium, in an amount corresponding to a daily dose of about 2 to 80 mg of atomic selenium equivalent, i.e about 0.025 to 1 mg/kg, for the production of a drug for treating severe systemic inflammatory response syndrome or any state corresponding to a severe acute attack of an inflammatory pathology causing an exacerbation of cytokine secretion, said molecule comprising selenium being selected among a selenium salt, selenoprotein P, selenocysteine, selenomethionine, selenoglutathione, selenomethyl, selenocysteine, dimethylselenoxide, selenocystamine and setenated yeasts.

2. Use of at least one molecule comprising selenium for the production of a drug for treating severe systemic inflammatory response syndrome, in an amount corresponding to a daily dose of about 2 to 80 mg of atomic selenium equivalent, i.e. about 0.025 to 1 mg/kg, at the beginning of the treatment, then at a daily dose of about 0.5 to 2 mg of atomic selenium equivalent, said molecule comprising selenium being selected among a selenium salt, selenoprotein P, selenocysteine, selenomethionine, selenoglutathione, selenomethyl, selenocysteine, dimethylselenoxide, selenocystamine and selenated yeasts.

3. Use according to one of claims 1 and 2, **characterised in that** the selenium salt is selected among a selenite or selenate of inorganic selenium.

4. Use according to one of claims 1 to 3 wherein the drug is intended for treating severe acute infectious states, such as peritonitis, pneumopathies, meningitis or bacterial septicemias in a septic shock state.

5. Use according to one of claims 1 to 3 for treating severe infectious states whether of bacterial, parasitic, fungal, or viral origin and, in general, any condition accompanied by a significant immuno-inflammatory reaction with in particular an increase in circulating cytokines, but also more localized conditions such as an attack of rheumatoid polyarthritis.

6. Use of the drug according to any one of the preceding claims for treatment in man or animals, the doses per kg being, in animals, modulated according to the 50% lethal dose (LD 50) of the species in comparison with that of the human species.

7. Use according to any one of the preceding claims wherein the drug is produced so as to give a daily dose of about 2 to 80 mg of atomic selenium equivalent, i.e about 0.025 to 1 mg/kg, during the first day, and optionally the second, third and fourth days of treatment.

8. Use according to any one of the preceding claims wherein the drug is produced so as to give a daily dose of about 0.5 to 2 mg of atomic selenium equivalent, for 1 to 20 days during the subsequent treatment.

9. Use according to any one of the preceding claims wherein one of the molecules comprising selenium is sodium selenite.

10. Use according to any one of the preceding claims wherein several molecules comprising selenium are used simultaneously to modulate more precisely different compartments of the systemic inflammatory reaction.

11. Use according to any one of the preceding claims, **characterized in that** the drug is in a form which may be administered by the parenteral route, preferably by intravenous, and also by subcutaneous, intramuscular, and also by intraperitoneal, enteral or oral routes, and advantageously in an injectable or perfusable pharmaceutical form or for enteral administration.

12. Use according to any one of the preceding claims, **characterized in that** the drug comprises at least one associated non-selenium compound inhibiting, or reducing the consequences of oxidative metabolism or inhibiting the inflammatory reaction.

13. Use according to claim 12, **characterized in that** the associated non-selenium compound which inhibits oxidative metabolism is selected from a glutathione precursor, an iron chelator, a copper chelator, copper, zinc, vitamin E and optionally vitamin C.

14. Use according to claim 12, **characterized in that** the compound inhibiting the inflammatory reaction is gold.

15. Use according to any one of the preceding claims, **characterized in that** the drug comprises an essential oligo-element other than selenium or those cited above (Cu, Zn).

16. Pharmaceutical composition **characterized in that** it comprises an amount of molecule or molecules comprising selenium corresponding to a daily dose of about 2 to 80 mg of atomic selenium equivalent, i.e. about 0.025 to 1 mg/kg, and pharmaceutically acceptable excipients, said molecule comprising selenium being selected amoung a selenium salt, selenoprotein P, selenocysteine, selenomethionine, selenoglutathione, selenomethyl, selenocysteine, dimethylselenoxide, selenocystamine and selenated yeasts.

17. Pharmaceutical composition according to claim 16, **characterized in that** the selenium salt is selected amoung a selenite or selenate of inorganic selenium.

18. Pharmaceutical composition according to one of claims 16 and 17, **characterized in that** it comprises at least one associated non-selenium compound inhibiting or reducing the consequences of oxidative metabolism or inhibiting the inflammatory reaction.

19. Pharmaceutical composition according to claim 18, **characterized in that** the associated non-selenium compound is selected from vitamin E and optionally vitamin C, a glutathione precursor, an iron chelator, a copper chelator, copper, or zinc.

20. Pharmaceutical composition according to of claims 16 and 17, **characterized in that** the compound inhibiting the inflammatory reaction is gold.

21. Composition according to any one of claims 16 to 20, **characterized in that** it comprises an essential oligo-element other than selenium or those cited above (Cu, Zn).

22. Composition according to any one of claims 16 to 21, **characterized in that** it is in an injectable or perfusable form for parenteral administration, preferably intravenous (also subcutaneous or intramuscular), but also intraperitoneal, enteral or oral.

23. Composition according to any one of claims 16 to 21, **characterized in that** it is in the form of a perfusion comprising between about 1.3 and 800 mg of atomic selenium equivalent per litre.
